# EUROPEAN PATENT APPLICATION

(11) **EP 2 014 765 A2**
(43) Date of publication of application: **14.01.2009**
(21) Application number: 08011623.9
(22) Date of filing: 26.06.2008
(51) Int. Cl.: C12N 1/20, C12P 17/12, A61K 31/495, A61P 35/00, C12R 1/01

(54) **A novel species of acrocarpospora, a method of preparing iodinin, and the uses of iodinin**

(30) Priority: 13.07.2007 US 959553 P
(71) Applicant: Food Industry Research and Development Institute, Hsinchu City 300 (TW)
(72) Inventor: Tseng, Min, Hsinchu City 300 (TW); Cheng, Ming-Jen, Hsinchu City 300 (TW); Yang, Shu-Feng, Hsinchu City 300 (TW); Yuan, Gwo-Fang, Hsinchu City 300 (TW); Yu, Li-Wen, Hsinchu City 300 (TW); Wu, Wen-Jung, Hsinchu City 300 (TW)
(74) Representative: Becker Kurig Straus

(57) **Abstract**

The present invention provides a novel species of *Acrocarpospora* gen. that is capable of producing iodinin. The invention also provides a method of preparing iodinin, and uses of iodinin in inducing cytotoxicity and treating cancers.

## Description

### FIELD OF THE INVENTION

The present invention relates to a novel species of *Acrocarpospora* gen. that is capable of producing iodinin, a method of preparing iodinin, and uses of iodinin in inducing cytotoxicity in cells and treating cancers.

### BACKGROUND OF THE INVENTION

Iodinin, 1, 6-phenazinediol 5, 10-dioxide, is a purple, red-glinting pigment, originally found to cover the colonies of isolated *Chromobacterium iodinum* on suitable solid media. It has been found to be one of the phenazine compounds and is known to be produced by microorganisms. Iodinin has been of great interest in the antibiotic field since it can be converted to a pharmaceutically valuable antibiotic, myxin, and a lot of effort has been put into developing a suitable process for its manufacture, such as the one disclosed in U.S. Pat. No. 3,663,373.

Along with the above anti-bacterial property, U.S. Pat. No. 3,764,679 reported that iodinin can be used as an effective antihypertensive agent for lowering blood pressure in a hypersensitive patient.

The genus *Acrocarpospora* was first disclosed by Tamura *et al.* (2000), and said genus currently comprises the following three species: *A. corrugatum, A. macrocephala* and *A. pleiomorpha*. The members of the genus are aerobic, Gram-positive, no-acid-fast, non-motile organisms that form a branched substrate mycelium. Non-fragmentary substrate mycelia are present. Spherical and club-shaped structures, which contain coiled spore chains, are borne on the tips of the aerial mycelium. The spores are oval or short rod-like with a smooth surface and non-motile. The organism contains *meso*-diaminopimelic acid (A₂pm), and madurose, glucose, arabinose and rhamnose are detected in whole-cell sugars (type B) (Lechevalier & Lechevalier, 1970). The major cellular fatty acids are iso-C_{16:0}, C_{16:0}, C_{17:0}, and 10-methyl-C_{17:0}, and phosphatidylethanolamine is present as a diagnostic phospholipid. The major menaquinones are MK-9(H₄) and MK-9(H₂), and mycolic acids are absent in the culture. The members of the genus have a DNA G+C content of 68-69 mol%.

In the present invention, we discovered a novel species of *Acrocarpospora* gen. that is capable of producing iodinin. We also discovered a new function of iodinin in inducing cytotoxicity in mammalian cells which has never been reported.

### SUMMARY OF THE INVENTION

One object of the invention is to provide a novel isolated *Acrocarpospora* strain, which is capable of producing iodinin. The isolated *Acrocarpospora* strain is preferably strain 04107M-2, which was deposited with the ATCC under the accession number PTA-8410, or a variant or mutant thereof.

Another object of the invention is to provide a method for the preparation of iodinin wherein the iodinin is produced by incubating the isolated *Acrocarpospora* strain of the invention. The iodinin produced may be further isolated or purified.

Another object of the invention is to provide a composition for inducing cytotoxicity in a cell of an animal which comprises an effective amount of iodinin. In one aspect, the animal is a mammal, and preferably a human. In another aspect, the cell is a cancer cell or cell lines such as AGS, HeLa, HepG2, and MCF-7.

A further object of the invention is to provide a use of iodinin in the manufacture of a medicament for inducing cytotoxicity in a cell of an animal. In one aspect, the animal is a mammal, and preferably a human. In another aspect, the cell is a cancer cell or cell lines such as AGS, HeLa, HepG2, and MCF-7.

A further object of the invention is to provide a method for inducing cytotoxicity in a cell of an animal which comprises contacting the cell with iodinin. In one aspect, the animal is a mammal, and preferably a human. In another aspect, the cell is a cancer cell or cell lines such as AGS, HeLa, HepG2, and MCF-7.

Another object of the invention is to provide a pharmaceutical composition for treating cancers in an animal which comprises an effective amount of iodinin. In one aspect, the animal is a mammal, and preferably is a human.

Another object of the invention is to provide a use of iodinin in the manufacture of a medicament for treating cancers.

Still another object of the invention is to provide a method for treating cancers in an animal which comprises administering an effective amount of iodinin to the animal in need of such treatment. In one aspect, the animal is a mammal, and preferably a human.

### BRIEF DESCRIPTION OF THE DRAWINGS

Figure 1 shows the scanning electron micrograph of the novel strain of the invention, 04107M-2, grown on HV agar at 28°C for 14 days. (Bar represents 1.5)µm.)

Figure 2 shows the growth of the strain of the invention, 04107M-2, on an oatmeal agar, on which reddish crystals with golden luster can be observed.

Figure 3 shows the Neighbour-joining tree on the basis of an almost complete sequence of 16S rDNA. The tree shows the phylogenetic position of the strain 04107M-2 within the *Acrocarpospora* species. The numbers at nodes indicate the percentages of 1000 bootstrap resamplings. Only the values over 50% are given. (Bar represents 0.01 substitutions per nucleotide position.)

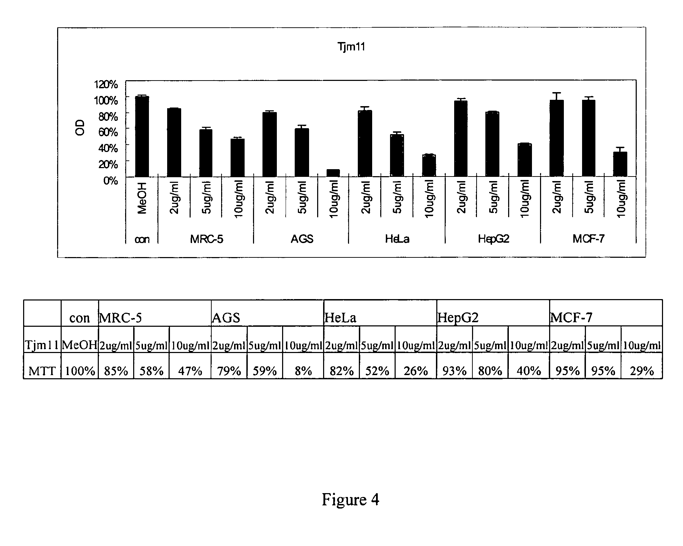

Figure 4 shows the cytotoxic effect of iodinin on the growth of various tumor cells. Cell growth inhibitory effects were determined by an MTT assay after treating the cell lines with 2, 5, and 10 µg/ml of iodinin for 72 hours. Mean ± SE of four independent experiments are shown. The cytotoxic activity of the compound iodinin was tested *in vitro* in MRC-5, AGS, HeLa, HepG2 and MCF-7 cell lines. The compound iodinin was found to exhibit a significant cytotoxic effect given that the percentages of cell survival for MRC-5, AGS, HeLa, HepG2 and MCF-7 at the iodinin concentration of 10 µg/ml are 47%, 8%, 26%, 40% and 29%, respectively.

### DETAILED DESCRIPTION OF THE INVENTION

### Definitions

Unless otherwise defined herein, scientific and technical terms used in connection with the present invention shall have the meanings that are commonly understood by those of ordinary skill in the art. The meanings and scope of the terms should be clear; however, in the event of any latent ambiguity, definitions provided herein take precedent over any dictionary or extrinsic definitions.

Generally, the methods and techniques of the present invention are performed according to conventional methods well known in the art and as described in various general and more specific references that are cited and discussed throughout the present specification, unless otherwise indicated. The nomenclature used in connection with and the laboratory procedures and techniques of the invention and medicinal and pharmaceutical chemistry described herein are those well known and commonly used in the art.

The term "isolated" or "isolation" means that the material is removed from its original environment (e.g., the natural environment if it is naturally existing). The term "isolated" does not necessarily reflect the extent to which the material has been purified.

The term "mutant" or "variant" is meant to encompass any microorganism whose total cellular genetic composition has been altered, for example, by chemical mutagenesis, spontaneous mutation, genetic engineering, transformation, or transfection, such that its physical or biochemical properties are affected. However, said variant or mutant should have all the identifying characteristics of the strain 04107M-2 deposited with the ATCC under accession number PTA-8410.

The term "whole broth culture" refers to a liquid culture containing both cells and media.

The term "treating" or "treatment" means the prevention or reduction of severe symptoms or effect of a pathological condition, including prolonging life expectancy. In the context of cancer therapy, treatment may include: prevention of tumor growth, reduction of tumor size, enhanced tumor cell death, or increased apoptosis in the tumor.

The term "effective amount" means an amount sufficient to effect beneficial or desired results.

The term "composition" means a combination of an active agent and another compound, carrier or composition, inert (for example, a detectable agent or label or liquid carrier) or active, such as an adjuvant.

The term "cytotoxicity" refers to both cell death and toxicity resulting in cell stasis, e.g., a loss of the ability to grow, as well as to apoptosis.

The term "mammals" means any class of higher vertebrates that nourish their young with milk secreted by mammary glands, including, for example, humans, rabbits, and monkeys.

### Acrocarpospora Strain

One object of the invention is to provide an isolated strain of *Acrocarpospora*. The strain of the present invention can be isolated from a natural source, e.g., a soil sample collected from Taitung County, Taiwan. The isolated strain of the present invention is capable of producing iodinin and has the following characteristics. The isolate strain displays substrate mycelia, and sporangia are borne on the aerial mycelia. The isolate strain contains *meso*-diaminopimelic acid in its peptidoglycan. Glucose, madurose, and rhamnose are the whole-cell sugars of the strain. The only phospholipid found is phosphatidylethanolamine (PE), and the predominant menaquinones are MK-9(H₄) and MK-9(H₂). Mycolic acids are not detected. Major cellular fatty acids are iso-C_{16:0} (19.6 %), C_{17:0} (16.84 %) and 10-methyl-C_{17:0} (26.51 %), and the DNA G+C content is 72.40 mol%. On the basis of the phenotypic and genotypic data, we proposed that strain 04107M-2 should be classified as a novel species of the genus *Acrocarpospora,* namely *Acrocarpospora punica* sp. nov. It was also found that a major purple, red-glinting compound, iodinin, could be obtained from the EtOAc extract of the submerged whole broth culture, which provides a new method of preparing iodinin.

Also contemplated within the scope of this invention is a strain of *Acrocarpospora punica* 04107M-2, which was deposited with the American Type Culture Collection 10801 University Blvd., Manassas, VA 20110-2209, U.S.A., on 3 May 2007 in accordance with the Budapest Treaty and assigned accession number PTA-8410. It was also deposited with the Bioresource Collection and Research Center, Taiwan, on 15 January 2007 (accession number BCRC 910341). Mutants or variants derived from the deposited strains (obtained by conventional or recombinant methods), as well as any *Acrocarpospora punica* strain which has characteristics that are identical to the deposited strain are also within the scope of this invention.

### Production of Iodinin

All *Acrocarpospora punica* strains of the invention, including the deposited strain and its mutants, can be used to produce iodinin. According to the present invention, the iodinin may be produced by incubating the *Acrocarpospora punica* strain of this invention in a suitable medium under a suitable condition to obtain a whole broth culture containing iodinin. The medium used in the present invention includes any media used to cultivate *Acrocarpospora*, such as HV medium, or any other media that can provide carbon and nitrogen sources and any components which are essential for synthesizing iodinin. The *Acrocarpospora punica* strain of the invention may be incubated at a temperature of about 20 to 30 °C, preferably about 28°C, for a period of about 1 to 3 weeks, preferably for about2 weeks.

According to the present invention, the iodinin in the whole broth culture may be further isolated or purified. The isolation or purification may be performed by any technologies known in the art. For example, the iodinin can be extracted by a solvent, such as EtOAc, CH₂Cl₂, or a mixture thereof.

### Uses of Iodinin

The iodinin obtained from the novel strain of the present invention was assessed in order to identify its properties. Surprisingly, upon conducting an MTT assay, it was found that iodinin exhibits cytotoxic effects against cell lines including tumor cell lines such as AGS, HeLa, HepG2, and MCF-7. In other words, iodinin is capable of inducing cytotoxicity in cells, and thus is capable of killing cells, including cancer cells. Since iodinin has only been reported for its anti-bacterial property and its use as an effective antihypertensive agent for lowering blood pressure in a hypersensitive patient, the invention provides a new use of iodinin in inducing cytotoxicity in animal cells.

On the basis of the fact that iodinin is capable of killing cancer cells, the invention further provides a method of treating cancer, comprising administering to an animal in need of such treatment an effective amount of iodinin in order to induce cytotoxicity in the cancer cells. The animal is preferably a mammal, and more preferably is a human.

The cancers that can be treated in the present invention include, but are not limited to, cervical cancer, liver cancer, gastric cancer, and breast cancer.

The iodinin used in the present invention can be obtained from any conventional sources. Examples of the conventional sources include, but are not limited to, *Chromobacterium iodinum, Nocardiopsis dassonvillei*, and *Acidithiobacillus ferrooxidans* (Ceskova et al., 2002). According to the present invention, the iodinin may also be obtained from the preparation process of the present invention as stated above.

The compound iodinin can be formulated as pharmaceutical compositions and administered to an animal, including a human patient, in a variety of forms adapted to the chosen route of administration. The compound is preferably administered in combination with a pharmaceutically acceptable carrier, and can be combined with or conjugated to specific delivery agents.

The compound can be administered by known techniques, e.g., oral, parental (including subcutaneous injection, intravenous, intramuscular, intrasternal or infusion techniques), by inhalation spray, topical, by absorption through a mucous membrane, or rectal, in dosage unit formulations containing one or more conventional non-toxic pharmaceutically acceptable carriers, adjuvants or vehicles. The pharmaceutical composition of the invention can be in the form of suspensions or tablets suitable for oral administration, nasal sprays, creams, and sterile injectable preparations, such as sterile injectable aqueous or oleagenous suspensions or suppositories.

For oral administration as a suspension, the composition can be prepared according to techniques well known in the art of pharmaceutical formulation. The composition may contain microcrystalline cellulose for imparting bulk, alginic acid or sodium alginate as a suspending agent, methylcellulose as a viscosity enhancer, and sweeteners or flavoring agents. As immediate release tablets, the composition may contain microcrystalline cellulose, starch, magnesium stearate and lactose or other excipients, binders, extenders, disintegrants, diluents, and lubricants known in the art.

For administration by inhalation or aerosol, the composition can be prepared according to techniques well known in the art of pharmaceutical formulation. The composition can be prepared as solutions in saline, using benzyl alcohol or other suitable preservatives, absorption promoters to enhance bioavailability, fluorocarbons, or other solubilizing or dispersing agents known in the art.

For administration as injectable solutions or suspensions, the composition can be formulated according to techniques well known in the art, using suitable dispersing or wetting and suspending agents, such as sterile oils, including synthetic mono- or diglycerides, and fatty acids, including oleic acid.

For rectal administration as suppositories, the composition can be prepared by mixing it with a suitable non-irritating excipient, such as cocoa butter, synthetic glyceride esters or polyethylene glycols, which are solid at ambient temperatures, but liquefy or dissolve in the rectal cavity to release the drug.

Preferred administration routes include oral and parenteral, as well as intravenous, intramuscular or subcutaneous routes.

More preferably, the compound iodinin is administered parenterally, i.e., intravenously or intraperitoneally, by infusion or injection. In one embodiment of the invention, the compound can be administered directly to a tumor by tumor injection. In another embodiment of the invention, the compound can be administered using systemic delivery by intravenous injection.

Solutions or suspensions of the compound can be prepared in water, or isotonic saline (PBS), and optionally mixed with a nontoxic surfactant. Dispe rsions can also be prepared in glycerol, liquid polyethylene, glycols, DNA, vegetable oils, triacetin and mixtures thereof. Und er ordinary conditions of storage and use, these preparations may contain a preservative to prevent the growth of microorganisms.

The pharmaceutical dosage form suitable for injection or infusion use may include sterile, aqueous solutions, dispersions, or sterile powders comprising an active ingredient which are adapted for the extemporaneous preparation of sterile injectable or infusible solutions or dispersions. The final dosage form should be sterile, fluid and stable under the conditions of manufacture and storage. The liquid carrier or vehicle can be a solvent or liquid dispersion medium comprising, for example, water, ethanol, a polyol such as glycerol, propylene glycol, or liquid polyethylene glycols, and the like, vegetable oils, nontoxic glyceryl esters, and suitable mixtures thereof. The proper fluidity can be maintained, for example, by the formation of liposomes, by the maintenance of the required particle size, in the case of dispersion, or by the use of nontoxic surfactants. The prevention of the action of microorganisms can be accomplished by various antibacterial and antifungal agents, for example, parabens, chlorobutanol, phenol, sorbic acid, thimerosal, and the like. In many cases, it will be desirable to include isotonic agents, for example, sugars, buffers, or sodium chloride. Prolong ed absorption of the injectable compositions can be brought about by the inclusion in the composition of agents delaying absorption such as aluminum monosterate hydrogels and gelatin.

Sterile injectable solutions are prepared by mixing the conjugates in the required amount in the appropriate solvent with various other ingredients as enumerated above, and filter sterilization is then conducted, as required. In the case of sterile powders for the preparation of sterile injectable solutions, the preferred methods of preparation are vacuum-drying and freeze-drying techniques, which yield a powder of the active ingredient plus any additional desired ingredient present in the previously sterile-filtered solutions.

When used *in vivo* to kill cancer cells, the administered dose is the amount that can produce the desired effect, such as the amount sufficient to reduce or eliminate tumors. Appropriate amounts can be determined by those skilled in the art, using known methods and relationships, from the in vitro data provided in the following examples.

The effective dose to be administered will vary with conditions specific to each patient. In general, factors such as the disease burden, tumor location (exposed or remote), host age, metabolism, sickness, prior exposure to drugs, and the like contribute to the expected effectiveness of a drug. One skilled in the art will use standard procedures and patient analysis to calculate the appropriate dose, extrapolating from the data provided in the following examples.

In addition, the composition of the invention can be administered in combination with other anti-tumor therapies.

All publications, patents, and patent documents described herein are incorporated by reference as if fully set forth. The invention described herein can be modified to include alternative embodiments. All such obvious alternatives are within the scope of the invention, as claimed below.

### Examples

The following examples are provided to aid those skilled in the art in practicing the present invention. Even so, the examples should not be construed so that they unduly limit the present invention, as modifications to and variations in the embodiments discussed herein may be made by those having ordinary skill in the art without departing from the spirit or scope of the present invention.

### A. Isolation of an Acrocarpospora Strain, Strain 04107TM-2

Strain 04107M-2 was isolated from a soil sample collected in Taitung County, Taiwan, by using HV agar (Hayakawa & Nonomura, 1987), and was then incubated at 28°C for 4 weeks. The strain was maintained on oatmeal agar and a suspension of spores or mycelia fragments of the strain in a broth containing 20% (vol/vol) glycerol was stored at -20°C.

### B. Characterization of the Strain 04107TM-2

### (1) Morphological Characteristics

The morphological characteristics of the strain 04107M-2 were observed by scanning electron microscope (S-450, Hitachi, Tokyo) following an incubation of said strain on HV agar at 28°C for 14 days, a fixation by 4% osmium tetraoxide solution, and then serial dehydrations with ethanol and acetone,and a critical point drying.

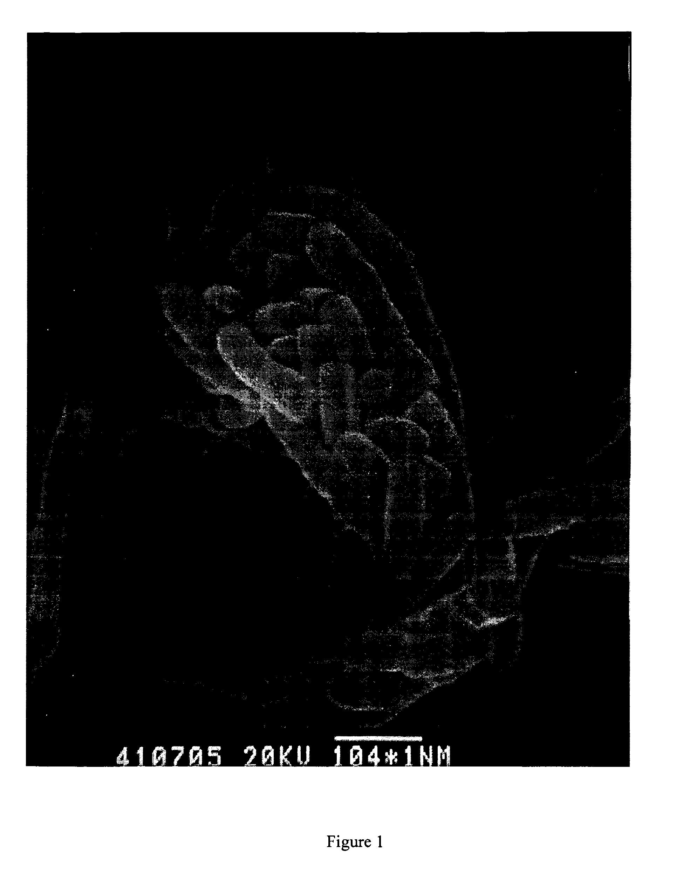

It was found that strain 04107M-2 produces branched and non-fragmented substrate mycelia, and club-shaped structures are borne on the tips of the aerial mycelium. The spores are non-motile, rod-like and smooth-surfaced, as shown in Figure 1.

### (2) Physiological Characteristics

All physiological tests were performed at 28°C. Growth temperature, hydrolysis of aesculin, casein, hypoxanthine, xanthine, adenine, and L-tyrosine, and production of amylase, nitrate reductase, and urease were detected by the methods and procedures described by Gordon *et al.* (1974).

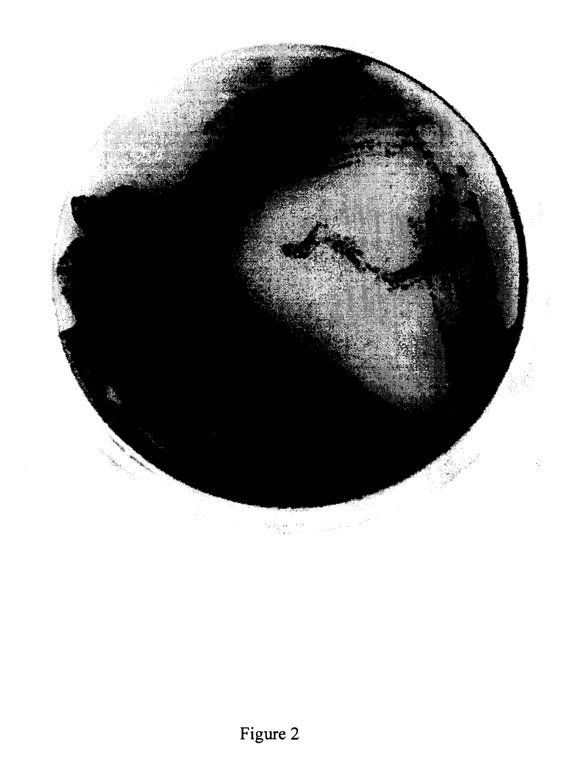

The growth of the strain on various media was poor, and reddish crystals with golden luster were observed on the oatmeal agar, as shown in Figure 2. No soluble pigment was found to be produced in any of the tested media. The results of the physiological tests are shown in Table 1.

**Table 1. The physiological characteristics of the strain 04107M-2**

| **Characteristics** | **Reaction** |
|---|---|
| Growth temperature (°C) | 20-30 |
| | |
| Decomposition of: | |
| Adenine | - |
| Aesculin | + |
| Casein | + |
| Hypoxanthine | - |
| L-tyrosine | - |
| Xanthine | - |
| Production of: | |
| Amylase | - |
| Melanin | - |
| Nitrate reductase | - |
| Urease | + |

| | |
|---|---|
| *+: positive reaction, -: negative reaction, +/-: doubtful reaction | |

### (3) Biochemical Characteristics

Biomass for chemotaxonomic studies was prepared after growing the strain in shaking flasks (125 rpm/min) with YS broth (10.0g of yeast extract and 10.0g of starch in 1.0L of distilled water of pH 7.0) at 28°C for 14 days. The isomer of diaminopimelic acid and sugars in the whole-cell hydrolysates were determined by the method described by Hasegawa *et al.* (1983). The presence of mycolic acids was examined via Thin Layer Chromatography (TLC) by following the method disclosed in Minnikin *et al.* (1975), and phospholipids were extracted and identified by following the method disclosed in Minnikin *et al.* (1984). Men aquinones were extracted and purified by the method disclosed in Collins *et al.* (1977) and then analyzed by HPLC (Model 600, Waters) with a Nova-Pak C18 column. For quantitative analysis of the cellular fatty acid contents, strain 04107M-2 was cultivated using TSB medium at 28°C in a shaking incubator at 125 r.p.m. for 7 days. Extracts of the methylated fatty acids were prepared according to the protocol provided by the manufacturer (Microbial ID Inc., U. S. A.).

It was found that *meso*-A₂pm, madurose, rhamnose and glucose are contained in the whole-cell hydrolysates of the strain 04107M-2. Predominant menaquinones were found to be MK-9(H₄) and MK-9(H₂), and mycolic acid was not detected. However, phosphatidylethanolamine (PE) was detected. The major fatty acid methyl esters are iso-C_{16:0} (19.6%), C_{17:0} (16.84%) and 10-methylC_{17:0} (26.51%), and the G+C content of the DNA is 72.40 mol%.

### (4) Phylogenetic Characteristics

For the extraction of the DNA to be used for sequencing the 16S rRNA gene, the strain 04107M-2 was grown in YS broth at 28°C for 14 days. Cells were removed from the broth using a pipette tip and the total DNA was extracted using the QIAGEN^{®} Genimic DNA Kit. The G+C content of the DNA was determined by the HPLC method disclosed in Tamaoka & Komagata (1984). The 16S rRNA gene was PCR-amplified using the methods provided by Nakajima *et al.* (1999) and directly sequenced on an ABI model 3730 automatic DNA sequencer of BigDye Terminator V3.1 Kit (Applied Biosystems). Phylogenetic analysis was performed using the software packages PHYLIP and MEGA (Molecular Evolutionary Genetics Analysis) version 2.1 (Kumar *et al.*, 2001) after multiple alignments of the data using CLUSTALX (Thompson *et al.*, 1997). Evolutionary distances were calculated (distance options according to the Kimura two-parameter model; Kimura, 1980) and the sequences were clustered with the neighbor-joining method described by Saitou & Nei, 1987. Bootstrap analysis was performed to evaluate the tree topology of the neighbor-joining data by performing 1000 resamplings. DNA-DNA hybridization was carried out according to the method disclosed in Ezaki *et al.* (1989).

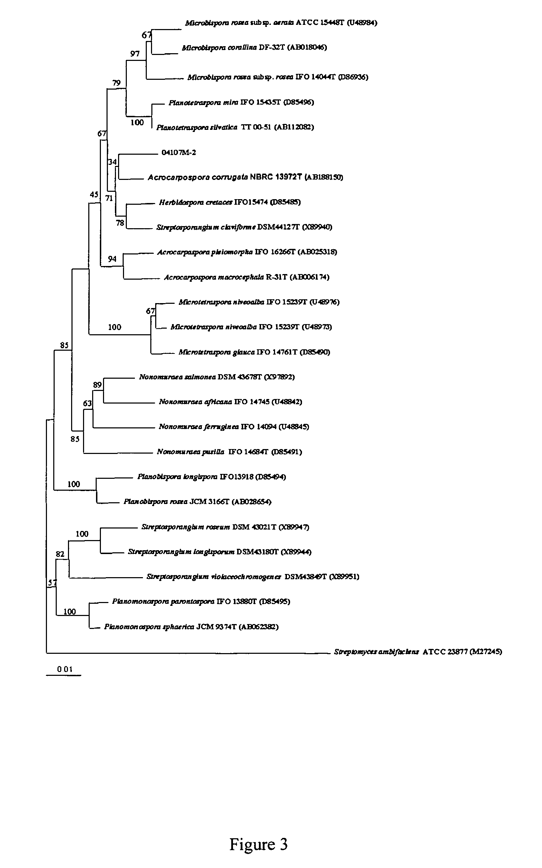

Following the aforementioned methods, the almost-complete sequence (1511 nt) of the 16S rRNA gene of the strain 04107M-2 was determined. Prelimi nary comparison of the sequence against the GenBank database revealed high sequence similarity values with certain members of the genus *Acrocarpospora*. The phylogenetic tree obtained on the basis of the sequence of the 16S rRNA gene of the strain 04107M-2, other valid published *Acrocarpospora* species and related species is shown in Figure 3. Binary similarity values range between 96.5% (*A. pleiomorph* IFO 16266^{T}) and 98.2% (*A. corrugata* NBRC 13972^{T}). DNA-DNA hybridization rates of the new isolate 04107M-2 to its closest type of strains, *A. corrugata* BCRC 16357^{T}, *A. macrocephala*, and *A. pleiomorpha*, are 2.9%, 0.4%, and 1.0%, respectively (see Table 2).

**Table 2. DNA hybridization rates among species of Acrocarpospora**

| Probe | 04170M-2(%) | *A. corrugata*^{T} (%) |
|---|---|---|
| 04107M-2 | 100.0 | 14.9 |
| *A. corrugata^{T}* | 2.9 | 100.0 |
| *A. macrocephala^{T}* | 0.4 | 4.5 |
| *A. pleiomorpha^{T}* | 1.0 | 8.6 |

On the basis of the results of the DNA-DNA relatedness study ( <70% ) , it is clear that the strain 04107M-2 and these two species are different species (Wayne *et al.*, 1987).

The distinctiveness of the isolate also comes from the phenotypic evidence compared with the nearest phylogenetic neighbours (see Table 2). On the basis of the phenotypic and genotypic characteristics, it is evident that the isolate should be classified as a new species of the genus *Acrocarpospora, Acrocarpospora punica* sp. nov., with the type strain 04107M-2.

### C. Compound Indentification of Iodinin

The whole broth (10 L) was extracted with EtOAc and concentrated under reduced pressure to provide EtOAc- (fr. A, 5 g) soluble fractions. The EtOAc (5 g) soluble fractions were chromatographed on an Si gel (0.25 kg) column, eluted with *n*-hexane and enriched with EtOAc to yield 15 fractions (fr. A1∼fr. A15). Fr. A3 (1.56 g) was subjected to a Si gel (30 g) column, eluted with *n*-hexane, and enriched with EtOAc to yield 11 fractions (fr. A3-1-fr. A3-11). Fr. A3-11 (21.0 mg) were purified by preparative TLC (n-hexane-EtOAc, 5:1) to furnish iodinin.

Following the aforementioned methods, a major purple, red-glinting compound (phenazine type), iodinin, was obtained, and its physicochemical characteristics are provided below:

Purple needles (benzene); mp 235~236 °C; IR (KBr) νₘₐₓ 3500 (OH) cm⁻¹; ¹H NMR (CDCl₃, 400 MHz) δ 7.15 (2H, dd, *J* = 7.8, 0.9 Hz), 7.71 (2H, dd, *J* = 7.8, 9.3 Hz), 8.02 (2H, dd, *J* = 9.3, 0.9 Hz), 14.70 (2H, s); EIMS *m*/*z* 244 [M]⁺ (90). Molecular formula C₁₂H₈N₂O₄.

### D. Cytotoxic Effect of Iodinin

The cytotoxic effect of iodinin was determined by the MTT assay described below:

MRC-5 (embryonal lung, normal, human, BCRC60023), AGS (Human gastric adenocarcinoma, BCRC 60102), HeLa (Human cervical epithelioid carcinoma, BCRC 60005), HepG2 (Human hepatoblastoma, BCRC 60177), and MCF7 (human breast adenocarcinoma, BCRC 60436) were purchased from BCRC, Food Industry Research and Development Institute, Taiwan.

MRC-5, HeLa and HepG2 cells were cultivated in Minimum essential medium (MEM, GIBCO) supplemented with 10% fetal bovine serum (FBS, GIBCO) and 1% penicillin/streptomycin (GIBCO). AGS cells were cultivated in Ham's F-12 medium (F-12, GIBCO) supplemented with 10% FBS and 1% penicillin/streptomycin. MCF7 cells were cultivated in MEM supplemented with 10% FBS, 1% penicillin/streptomycin and 10ug/ml human recombinant insulin (GIBCO). All cell lines were maintained in an incubator at 37 °C with humidified air containing 5% CO₂.

The cytotoxic activities of the compound, iodinin, against all cell lines were assayed by a modified MTT [3-(4,5-dimethylthiazole-2-yl)-2,5-diphenyltetrazolium bromide] (Merck) colorimetric method. For MRC-5, AGS, HeLa, HepG2 and MCF7 cells, 180µl of cell cultures were initiated at the concentrations of 5000, 3000, 1500, 3000, and 3000 cells/well in 96-well tissue culture plates (Falcon), respectively. Th e compound iodinin was dispensed to the established cultures at three concentrations (5µM, 2µM, and 1µM) in quadruplicate. A fter 3 days of incubation, the cell numbers for each of the cell lines were determined by the following MTT assay: 20 µl of 1 mg/ml of MTT were added to each well of the plates, and the plates were then incubated at 37°C for 5 hours. Form azan crystals were redissolved in 100 µl of DMSO (Merck) for 10 minutes via shaking, and the plates were read immediately on an ELISA reader (Bio-Tek) at a wavelength of 540 nm (Mosmann, 1983).

The results, as shown in Figure 4, revealed that the compound iodinin is capable of inducing a significant cytotoxic effect in all of the tested cell lines.

### REFERENCES

Bush, J. A., Long, B. H., Catino, J. J., Braduer, W. T., and Tomita, K. (1987). Production and biological activity of rebeccamycin, a novel antitumor agent. J. Antibiot. 40: 668-678.
Collins, M. D., Pirouz, T., Goodfellow, M. & Minnikin, D. E. (1977). Distribution of menaquinones in actinomycetes and corynebacteria. J Gen Microbiol 100, 221-230.
Ezaki, T., Hashimoto, Y., & Yabuuchi, E. (1989). Fluorometric deoxyribonucleic acid-deoxyribonucleic acid hybridization in microdilution wells as an alternative to membrane filter hybridization in which radioisotopes are used to determine genetic relatedness among bacterial strains. Int J Syst Bacteriol 39, 224-229.
Gordon, R. E., Barnett, D. A., Handerhan, J. E. & Pand, C. H. -N. (1974). Nocardia coeliaca, Nocardia autotrophica, and the nocardin strain. Int J Syst Bacteriol 24, 54-63.
Hasegawa, T., Takizawa, M. & Tanida, S. (1983). A rapid analysis for chemical grouping of aerobic actinomycetes. J Gen Appl Microbiol 29, 319- 322.
Hayakawa, M. & Nonomura, H. (1987). Humic acid-vitamin agar, a new medium for the selective isolation of soil actinomycetes. J Ferment Technol 65, 501-509.
Igarashi, M., Chen, W., Tsucluda, T., Umekita, M., Sawa, T., Naganawa, H., Hamada, M., and Takeuchi, T. (1995). 4'-Deacetyl-(-)-griseusins A and B, new naphthoquinone antibiotics from an actinomycete. J. Antibiot. 48: 1502-1505.
Kimura, M. (1980). A simple method for estimating evolutionary rates of base substitutions through comparative studies of nucleotide sequence. J Mol Evol 16, 111-120.
Kumar, S., Tamura, K., Jakobsen, I.B. & Nei, M. (2001). MEGA2: Molecular Evolutionary Genetics Analysis software. Bioinformatics 17, 1244-1245.
Lechevalier, M. P. & Lechevalier, H. A. (1970). Chemical composition as a criterion in the classification of aerobic actinomycetes. Int. J. Syst. Bacteriol. 20: 435-443.
Makajima, Y., Kitpreechavanich, V., Suznki, K. & Kudo, T. (1999). Microbispora corallina sp. nov, a new species of the genus Microbispora isolated from Thai soil. Int. J Syst. Bacteriol. 49: 1761-1767.
Minnikin, D. E., Alshamaony, L. & Goodfellow, M. (1975). Differentiation of Mycobacterium, Nocardia, and related taxa by thin layer chromatographic analysis of whole-cell methanolysates. J Gen Microbiol 88, 200-204.
Ssitou, N. & Nei, M. (1987). The neighbor-joining method: a new method for reconstructing phylogenetic trees. Mol Biol Evol 4, 406- 425.
Montaner, B., and Perez-Tomas, R. (2003). The prodigiosins: a new family of anticancer drugs. Curr. Cancer Drug Targets. 3: 57-65.Nagasawa, T., Fukao, H., Irie, H. and Yamada, H. (1984). Sakyomicins A, B, C and D: new quinone-type antibiotics produced by a strain of Nocardia. Taxonomy, production, isolation and biological properties. J. Antibiot. 37: 693-699.
Mosmann, T. (1983). Rapid colorimetric assay for cellular growth and survival: Application to proliferation and cytotoxicity assays. J. Immunol. Methods 65, 55-63.
P. Ceskova, Z. Zak, D. B. Johnson, O. Janiczek, and M. Mandi (2002). Formation of iodinin by a strain of Acidithiobacillus ferrooxidans grown on elemental sulfur. Folia Microbiol (Praha). 47 (1): 78-80.
Tamaoka, J. & Komagata, K. (1984). Determination of DNA base composition by reversed-phase high-performance liquid chromatography. FEMS Microbiol Lett 25, 125-128.
Tamura, T., Suzuki, S. & Hatano, K. (2000). Acrocarpospora gen. Nov., a new genus of the order Actinomycetales. Int J Syst Bacteriol 50, 1163-1171.
Thompson, J. D., Gibson, T. J., Plewniak, F., Jeanmougin, F. & Higgins, D. G. (1997). The Clustal X windows interface: flexible strategies for multiple sequence alignment aided by quality analysis tools. Nucleic Acids Research 24, 4876-4882.
Wayne, L. G., Brenner, D. J., Colwell, R. R. & 9 other authors (1987). International Committee on Systematic Bacteriology. Report of the ad noc committee on reconciliation of approaches to bacterial systematics. Int. J. Syst. Bacteriol. 37: 463-464.

## Claims

1. An isolated strain of *Acrocarpospora* capable of producing iodinin, said strain having the characteristics of producing reddish crystals with golden luster when growing on solid media.

2. The isolated strain of Claim 1, which has the characteristics of:
substrate mycelia displayed;
sporangia borne on the aerial mycelia;
*meso*-diaminopimelic acid contained in peptidoglycan;
glucose, madurose, and rhamnose being the whole-cell sugars;
phosphatidylethanolamine (PE) being the only phospholipids;
MK-9(H₄) and MK-9(H₂) being the predominant menaquinones;
mycolic acids not being detected;
iso-C_{16:0} (19.6 %), C_{17:0} (16.84 %) and 10-methyl-C_{17:0} (26.51 %) being the major cellular fatty acids; and
DNA G+C content being 72.40 mol%.

3. An isolated strain of *Acrocarpospora purica* which is designated as *Acrocarpospora purica* 04107M-2 and deposited with the ATCC under the accession number PTA-8410, or a variant or mutant thereof having the characteristics substantially identical to those of *Acrocarpospora purica* 04107M-2.

4. A method for preparing iodinin comprising incubating the isolated strain of any one of Claims 1 to 3.

5. The method of Claim 4, wherein the iodinin is further isolated or purified.

6. An *in vitro* method for inducing cytotoxicity in a cell of an animal comprising contacting the cell with iodinin.

7. The method of Claim 6, wherein the iodinin is produced by the isolated strain of any one of Claims 1 to 3.

8. The method of Claim 6, wherein the cell is a cancer cell.

9. The method of Claim 8, wherein the cancer cell is from one of the following cancer cell lines: AGS, HeLa, HepG2, and MCF-7.

10. The method of Claim 6, wherein the animal is a mammal.

11. The method of Claim 10, wherein the mammal is a human.

12. A pharmaceutical composition for treating cancers of an animal comprising an effective amount of iodinin.

13. The pharmaceutical composition of Claim 12, wherein the iodinin is produced by the isolated strain of any one of Claims 1 to 3.

14. The pharmaceutical composition of Claim 12, wherein the animal is a mammal.

15. The pharmaceutical composition of Claim 14, wherein the mammal is a human.

16. The pharmaceutical composition of Claim 12, wherein the cancer is treated by inducing cytotoxicity in cancer cells of the animal.

17. The pharmaceutical composition of Claim 16, wherein the cancer is selected from cervical cancer, liver cancer, gastric cancer, and breast cancer.

18. Use of iodinin in the manufacture of a medicament for treating cancers of an animal.

19. The use of Claim 18, wherein the iodinin is produced by the isolated strain of any one of Claims 1 to 3.

20. The use of Claim 18, wherein the animal is a mammal.

21. The use of Claim 20, wherein the mammal is a human.

22. The use of Claim 18, wherein the cancer is treated by inducing cytotoxicity in cancer cells of the animal.

23. The use of Claim 22, wherein the cancer is selected from cervical cancer, liver cancer, gastric cancer, and breast cancer.
